# EUROPÄISCHE PATENTSCHRIFT

(11) **EP 0 385 964 B1**
(45) Veröffentlichungstag und Bekanntmachung des Hinweises auf die Patenterteilung: **11.08.1993**
(21) Anmeldenummer: 90890007.9
(22) Anmeldetag: 09.01.1990
(51) Int. Cl.: C12M 1/40, G01N 27/416

(54) **Biosensoranordnung**
Biosensor array
Biocapteur

(30) Priorität: 27.01.1989 AT 170/89
(43) Veröffentlichungstag der Anmeldung: 05.09.1990
(73) Patentinhaber: AVL Medical Instruments AG, 8207 Schaffhausen (CH)
(72) Erfinder: Marsoner, Hermann, Dipl.-Ing. Dr., A-8153 Steinberg (AT); List, Helmut, Dipl.-Ing., A-8010 Graz (AT); Kontschieder, Heinz, Dipl.-Ing., A-8010 Graz (AT); Skrabal, Falko, Prof. Dr., A-8010 Graz (AT)
(74) Vertreter: Krause, Walter, Dr. Dipl.-Ing.

## Beschreibung

Die Erfindung betrifft eine Sensoranordnung zur Bestimmung einer Substratkonzentration in einem Probenmedium mit einer membranbedeckten, amperometrischen Enzymelektrode und einer Referenzelektrode, welche mit einer Durchflußmeßzelle in Kontakt stehen.

In vielen Bereichen der Medizin oder auch der Biotechnik kommt der exakten Bestimmung einer bestimmten Substratkonzentration in einem Probenmedium, beispielsweise der Glucosekonzentration in einer Perfusionslösung, große Bedeutung zu. Häufig werden dazu elektrochemische Sensoren verwendet, wobei an Hand der Bestimmung der ß-D-Glucose das Meßprinzip dieser Elektroden erläutert werden soll.

Nach gängiger Praxis sind amperometrische Verfahren mit konstanter Polarisationsspannung Up zur Substratbestimmung besonders gut geeignet. Bei diesem Verfahren wird innerhalb einer Membran, in welcher das Enzym Glucoseoxidase (GOD) immobilisiert ist, die ß-D-Glucose entsprechend folgender chemischen Reaktionsgleichung umgewandelt:

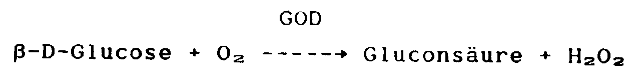

Das dadurch gebildete Wasserstoffperoxid kann durch anodische Oxidation an einer Platinelektrode detektiert werden: 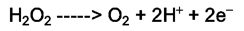 Up = 600 ... 700 mV

Die Größe des Sensorstromes ist von der Glucose-Konzentration der Probenlösung abhängig.

Die praktische Anwendung der amperometrischen Glucosebestimmung mit Hilfe von Enzymsensoren setzt nicht nur bestimmte chemische, sondern auch geometrische Randbedingungen voraus. Es muß zum Beispiel die Ausbildung der Anodenoberfläche hinreichend groß sein, damit eine ausreichende Sensorempfindlichkeit erreicht wird. Die Forderung nach einer großen Elektrodenoberfläche wird auch an die Referenzelektrode gestellt, über die der Stromkreis geschlossen wird. Eine großflächige, nichtpolarisierbare Referenzelektrode gestattet die ungehinderte Ableitung des Sensorstromes.

So ist beispielsweise aus der EP-AO 048 090 eine Sensoranordnung dereingangsgenannten Art bekanntgeworden, welche eine Durchflußmeßzelle aufweist, deren eine Seite von einer elektrochemischen Dünnschichtzelle bedeckt wird. Diese Zelle weist zwischen einer inneren und einer äußeren Membran eine Enzymschicht auf, wobei die innere Membran dicht an einer Halterung anliegt, welche in konzentrischer Anordnung eine Platinanode und eine Ag/AgCI-Referenzelektrode aufweist. Die Dünnschichtzelle wird mittels eines O-Ringes an der Halterung befestigt. Die äußere Membran dieser Dünnschichtzelle steht mit der in die Meßzelle eingebrachten Probe in Kontakt, wobei innerhalb von kurzer Zeit das zu messende Substrat und Sauerstoff in das innere der Dünnschichtzelle eindiffundieren und mit dem Enzym der Enzymschicht reagieren. Dabei wird Wasserstoffperoxid produziert, welches durch die innere Membran diffundiert und an der Platinanode die entsprechende Meßreaktion auslöst.

Es sind auch Elektrodenanordnungen bekannt, bei welchen das jeweilige Enzym direkt auf der Platinanode immobilisiert ist, wobei die eigentliche Enzymelektrode von einer kreisringförmigen Referenzelektrode umgeben und die gesamte Elektrodenanordnung von einer Kautschuk-Membran abgedeckt wird.

Nachteilig bei den bekannten Ausführungen ist, daß sie zum Teil kompliziert aufgebaut sind und relativ großvolumige, strömungsungünstige Probenkammern aufweisen, deren Abmessung direkt mit dem Durchmesser der Elektrodenanordnung zusammenhängt.

Aufgabe der Erfindung ist es, eine Sensoranordnung der eingangs genannten Art dahingehend weiter zu bilden, daß bei Aufrechterhaltung der Meßempfindlichkeit eine strömungsgünstige, kleinvolumige Durchflußmeßzelle realisiert werden kann, wobei die Gesamtanordnung möglichst einfach herstell bar sein soll.

Diese Aufgabe wird erfindungsgemäß dadurch gelöst, daß die Referenzelektrode außerhalb der Membranabdeckung der Enzymelektrode angeordnet ist und daß die maximale Ausdehnung der Durchflußmeßzelle normal zur Strömungsrichtung des Probenmediums im wesentlichen dem Durchmesser der sensitiven Schicht der Enzymelektrode entspricht. Es hat sich überraschenderweise gezeigt, daß mit membranfreien Referenzelektroden, welche beispielsweise in Strömungsrichtung des Probenmediums vor oder nach der eigentlichen Enzymelektrode angeordnet sind, ausgezeichnete Meßergebnisse erzielbar sind, wobei vorteilhafterweise sehr kompakte, strömungsgünstige Meßzellen möglich sind, deren Breite normal zur Strömungsrichtung im wesentlichen durch den Durchmesser der eigentlichen Enzymelektrode bestimmt wird. Durch die strömungsgünstige Ausbildung wird die Ablagerung von Probenbestandteilen in der Meßzelle minimiert und eine allfällige Reinigung erleichtert.

Eine besonders vorteilhafte Weiterbildung der Erfindung, durch welche das Meßzellenvolumen weiter verkleinert werden kann, sieht vor, daß die Durchflußmeßzelle zwei gegenüberliegende Deckflächen aufweist, daß in einer der Deckflächen die sensitive Schicht der Enzymelektrode und in der anderen Deckfläche die membranfreie Referenzelektrode, vorzugsweise eine Ag-Elektrode, liegt. Ein weiterer Vorteil bei extern angeordneten membranfreien Referenzelektroden besteht darin, daß in Sensoranordnungen welche austauschbare Elektroden aufweisen, nur die eigentliche Enzymelektrode ausgetauscht werden muß, wobei die separate, praktisch wartungsfreie Referenzelektrode in der Sensoranordnung verbleiben kann.

In vielen Meßsituationen muß neben der Substratkonzentration noch ein weiterer Parameter der Probenflüssigkeit bestimmt werden, beispielsweise deren elektrische Leitfähigkeit bzw. Impedanz, wobei im Sinne einer kompakten Ausführung der Sensoranordnung erfindungsgemäß vorgeschlagen wird, daß die Referenzelektrode Teil einer Elektrodenanordnung zur Impedanzmessung ist, wobei die zugehörige Gegenelektrode vorzugsweise in der der Referenzelektrode gegenüberliegenden Deckfläche der Durchflußmeßzelle angeordnet ist. Während die Enzymelektrode und auch die Referenzelektrode wie eingangs ausgeführt relativ großflächig ausgebildet sein sollten, reicht es für die Gegenelektrode einen dünnen Metallstift, vorzugsweise einen Silberdraht, vorzusehen, welcher in die Durchflußmeßzelle eintaucht. Aus Platzgründen empfiehlt sich beispielsweise eine - in Strömungsrichtung gesehene - Anbringung vor den sich gegenüberstehenden Enzym-und Referenzelektroden.

Eine Sensoranordnung, in welcher neben der Su bstratkonzentration auch die Leitfähigkeit bzw. Impedanz gemessen wird, kommt vor allem dann zur Anwendung, wenn Perfusionslösungen gemessen werden, die mit dem Gewebe eines Organismus in Kontakt gebracht wurden, wobei nur eine teilweise Equilibration der Perfusionslösung mit dem im Gewebe vorliegenden zu messenden Substrat zustande kommt. Das Ausmaß der Equilibration ist dann durch die sich ändernde Leitfähigkeit der Perfusionslösung bestimmbar, wodurch die eigentliche Substratkonzentration auch bei nur teilweiser Equilibration errechnet werden kann.

Es ist entsprechend einer Weiterbildung der Erfindung auch möglich, daß die Gegenelektrode der Elektrodenanordnung zur Impedanzmessung durch die Anode der Enzymelektrode gebildet ist. Für den Meßbetrieb ergeben sich dann prinzipiell zwei mögliche Vorgangsweisen. Entweder wird die Enzymkonzentration und die Impedanz alternierend gemessen, oder der Polarisationsspannung für die Enzymmessung wird eine Wechselspannung fürdie Impedanzmessung überlagert, wobei die beiden Signale dann elektronisch getrennt und ausgewertet werden.

Gerade bei Impedanzmessungen, aber auch im Hinblick auf die temperaturabhängigen Reaktionen im Enzymsensor ist es meist wichtig, eine Information über die Temperatur des Probenmediums zu erhalten. Eine für die Herstellung besonders einfache Weiterbildung der Erfindung ist in diesem Zusammenhang dadurch gegeben, daß in einer der Gegenelektrode koaxial gegenüberliegenden Bohrung im Gehäuse der Durchflußmeßzelle ein Temperatursensor, vorzugsweise ein NTC-Widerstand angeordnet ist. Es entsteht somit eine äußerst kompakte Durchflußzelle zur kontinuierlichen Bestimmung der Substratkonzentration, der Impedanz und der Temperatur eines Probenmediums.

Schließlich ist erfindungsgemäß vorgesehen, daß die Enzymelektrode als Glucosesensor ausgebildet ist. Das Enzym Glucoseoxidase kann dabei in an sich bekannter Art direkt auf der Platinanode oder in einer Trägermembran immobilisiert sein.

Die Erfindung wird nun anhand von Ausführungsbeispielen näher erläutert. Es zeigen:
Fig. 1 eine Sensoranordnung nach der Erfindung geschnitten nach der Linie I-I in Fig. 2,
Fig. 2 einen Schnitt nach der Linie 11-11 in Fig. 1, sowie
Fig. 3 eine Ausführungsvariante in einer Fig. 2 entsprechenden Schnittdarstellung.

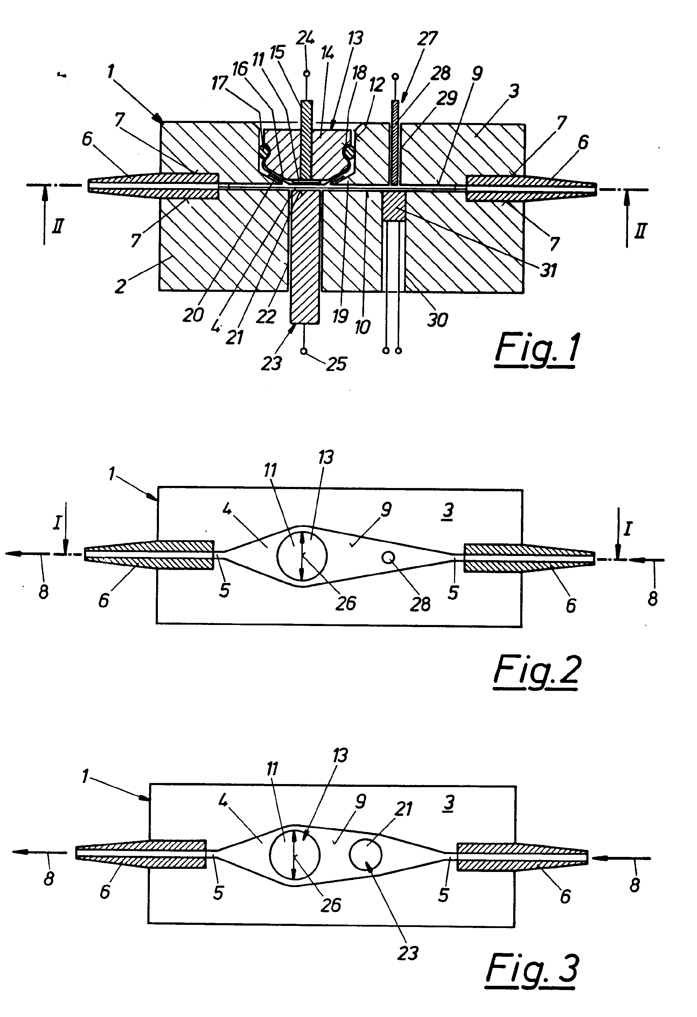

Die in Fig. 1 dargestellte Sensoranordnung weist ein zweigeteiltes Gehäuse 1 auf, dessen Unterteil 2 und Oberteil 3 in der Ebene der als Durchflußmeßzelle 4 ausgeführten Meßkammer zusammengeklebt sind. Die Meßzelle 4 läuft - wie aus Fig. 2 ersichtlich - sowohl zufluß- als auch abflußseitig in Kapillarbohrungen 5 aus, welche an Anschlußelemente 6 anschließen, die in Ausnehmungen 7 des Unterteils 2 und Oberteils 3 gehalten sind und beim Zusammenkleben beider Gehäuseteile eingeklebt werden. Die Durchflußrichtung des Probenmediums ist durch Pfeile 8 angedeutet.

Die Durchflußmeßzelle 4 weist zwei gegenüberliegende Deckflächen 9 und 10 auf, wobei in einer 9 die sensitive Schicht 11 der in einer Öffnung 12 des Oberteils 3 angeordneten Enzymelektrode 13 liegt.

Die Enzymelektrode 13 weisteine Halterung 14 auf, die zentrisch von einer Platinanode 15 durchsetzt wird. Meßzellenseitig wird das Ende der Platinanode 15 von der sensitiven Schicht 11 bzw. einer Enzymmembran kontaktiert, welche ihrerseits von einer äußeren Membran 16 abgedeckt ist. Die Membran 16 wird durch einen O-Ring 17, welcher in eine Ringnut 18 der Halterung 14 einrastet, gehalten. Der O-Ring 17 sorgt gleichzeitig für einen reibschlüssigen Sitz der Enzymelektrode 13 in der Öffnung 12 des Gehäuses 1. Meßzellenseitig ist ein an einem Absatz 19 der Öffung 12 anliegendes Dichtelement 20 vorgesehen, durch welches die Enzymelektrode gegen den Oberteil 3 des Gehäuses abgedichtet wird.

In der der Enzymelektrode 13 gegenüberliegenden Deckfläche 10 der Durchflußmeßzelle 4 befindet sich die Stirnfläche 21 der in einer Bohrung 22 des Unterteils 2 angeordneten Referenzelektrode 23. Die elektrischen Anschlüsse der Enzymelektrode 13 bzw. der Referenzelektrode 23 sind mit Bezugszeichen 24 bzw. 25 bezeichnet.

Durch die externe Anbringung der Referenzelektrode 23 ist es möglich, die Meßzelle 4 kleinvolumig und strömungsgünstig auszubilden, wobei die maximale Ausdehnung der Meßzelle 4 normal zur Strömungsrichtung des Probenmediums im wesentlichen dem Durchmesser 26 der sensitiven Schicht 11 der Enzymelektrode 13 entspricht.

Beide Meßzellenteile können als Spritzgußteil aus einem elektrisch isolierendem Material, beispielsweise aus Plexiglas ausgeführt sein; es ist jedoch auch möglich, die für die einzelnen Elektroden notwendigen Bohrungen bzw. Öffnungen mit entsprechenden Bohr- und Fräswerkzeugen herzustellen.

Der Equilibrierungsgrad von Perfusionslösungen kann dadurch bestimmt werden, daß die elektrische Impedanz zwischen zwei Metallelektroden die mit der Perfusionslösung in Kontakt stehen, gemessen wird. Durch die freiliegende externe Anordnung der Referenzelektrode 23 kann diese als Teil einer Elektrodenanordnung 27 für die Impedanzmessung verwendet werden. Die zugehörige Gegenelektrode 28, angeordnet in einer Bohrung 29 des Oberteils 3, endet in der der Referenzelektrode 23 gegenüberliegenden Deckfläche 9 der Meßzelle 4.

Es ist jedoch auch möglich, daß die Gegenelektrode 28 zur Impedanzmessung durch die Anode 15 der Enzymelektrode 13 realisiert wird, wodurch die Sensoranordnung weiter vereinfacht wird.

Des weiteren ist in einer der Gegenelektrode 28 koaxial gegenüberliegenden Bohrung 30 im Gehäuse 1 der Durchflußmeßzelle 4 ein Temperatursensor 31 angeordnet. Dazu wird vorzugsweise ein angeschliffener NTC-Widerstand verwendet. Somit kann mit dieser miniaturisierten Durchflußmeßzelle gleichzeitig die Substratkonzentration, der Equilibrierungsgrad und die Temperatur einer Probe bestimmt werden. Das Meßzellenvolumen beträgt beispielsweise nur 15 wl.

Fig. 3 zeigt, daß es in einer Ausführungsvariante der Erfindung auch möglich ist, die Enzymelektrode 13 und die membranfreie Referenzelektrode 23 in einer Deckfläche der Meßzelle 4 anzuordnen. Auch hier entsteht eine strömungsgünstige Meßzelle deren maximale Breite im wesentlichen dem Durchmesser 26 der sensitiven Schicht 11 der Enzymelektrode 13 entspricht.

## Patentansprüche

1. Sensoranordnung zur Bestimmung einer Substratkonzentration in einem Probenmedium mit einer membranbedeckten, amperometrischen Enzymelektrode und einer Referenzelektrode, welche mit einer Durchflußmeßzelle in Kontakt stehen, dadurch gekennzeichnet, daß die Referenzelektrode (23) außerhalb der Membranabdeckung der Enzymelektrode (13) angeordnet ist und daß die maximale Ausdehnung der Durchflußmeßzellen (4) normal zur Strömungsrichtung des Probenmediums im wesentlichen dem Durchmesser (26) der sensitiven Schicht (11) der Enzymelektrode (13) entspricht.

2. Sensoranordnung nach Anspruch 1, dadurch gekennzeichnet, daß die Durchflußmeßzelle (4) zwei gegenüberliegende Deckflächen (9, 10) aufweist, daß in einer der Deckflächen (9) die sensitive Schicht (11) der Enzymelektrode (13) und in der anderen Deckfläche (10) die membranfreie Referenzelektrode (23), vorzugsweise eine Ag-Elektrode, liegt.

3. Sensoranordnung nach Anspruch 1 oder 2, dadurch gekennzeichnet, daß die Referenzelektrode (23) Teil einer Elektrodenanordnung (27) zur Impedanzmessung ist, wobei die zugehörige Gegenelektrode (28) vorzugsweise in der der Referenzelektrode (23) gegenüberliegenden Deckfläche (9) der Durchflußmeßzelle (4) angeordnet ist.

4. Sensoranordnung nach Anspruch 3, dadurch gekennzeichnet, daß die Gegenelektrode (28) der Elektrodenanordnung (27) zur Impedanzmessung durch die Anode (15) der Enzymelektrode (13) gebildet ist.

5. Sensoranordnung nach Anspruch 3, dadurch gekennzeichnet, daß in einer der Gegenelektrode (28) koaxial gegenüberliegenden Bohrung (30) im Gehäuse der Durchflußmeßzelle (4) ein Temperatursensor (31), vorzugsweise ein NTC-Widerstand angeordnet ist.

6. Sensoranordnung nach einem derAnsprüche 1 bis 5, dadurch gekennzeichnet, daß die Enzymelektrode (13) als Glucosesensor ausgebildet ist.

## Claims

1. A sensor device for determining the concentration of a substrate in a sample medium, comprising a membrane-covered amperometric enzyme electrode and a reference electrode, both of which are in contact with a flow measuring cell, wherein the reference electrode (23) is positioned outside of the membrane cover of the enzyme electrode (13), and wherein the maximum extension of the flow measuring cell (4) normal to the flow direction of the sample medium is essentially the same as the diameter (26) of the sensitive layer (11) of the enzyme electrode (13).

2. A sensor device according to claim 1, wherein the flow measuring cell (4) has two opposite faces (9, 10), one of which (9) contains the sensitive layer (11) of the enzyme electrode (13) and the other one (10) the membrane-free reference electrode (23), preferably an Ag-electrode.

3. A sensor device according to claim 1 or 2, wherein the reference elctrode (23) constitutes part of an electrode arrangement (27) for impedance measurement, the corresponding complementary electrode (28) preferably being located in the surface (9) of the flow measuring cell (4) opposite of the reference electrode (23).

4. A sensor device according to claim 3, wherein the complementary electrode (28) of the electrode arrangement (27) for impedance measurement is formed by the anode (15) of the enzyme electrode (13).

5. A sensor device according to claim 3, wherein a temperature sensor (31), preferably an NTC resistor, is inserted into a bore (30) in the housing of the flow measuring cell (4), the said bore being coacial with and opposite of the complementary electrode (28).

6. A sensor device according to any of claim 1 to 5, wherein the enzyme electrode (13) is configured as a glucose sensor.

## Revendications

1. Capteur pour déterminer une concentration en substance dans un échantillon à l'aide d'une électrode à enzymes, ampérométrique, recouverte par une membrane et une électrode de référence, ces électrodes étant en contact avec une cellule de mesure à passage, capteur caractérisé en ce que l'électrode de référence (23) est placée à l'extérieur de la membrane de recouvrement de l'électrode à enzymes (13) et en ce que l'extension maximale des cellules à passage (4) perpendiculairement à la direction d'écoulement de l'échantillon correspond principalement au diamètre (26) de la couche sensible (11) de l'électrode à enzymes (13).

2. Capteur selon la revendication 1, caractérisé en ce que la cellule de mesure à passage (4) comporte deux surfaces de recouvrement (9, 10) opposées et en ce que dans l'une des surfaces de recouvrement (9) se trouve la couche sensible (11) de l'électrode à enzymes (13) et dans l'autre couche de recouvrement (10) se trouve l'électrode de référence (23) sans membrane, de préférence une électrode Ag.

3. Capteur selon la revendication 1 ou 2, caractérisée en ce que l'électrode de référence (23) fait partie d'un dispositif à électrodes (27) pour la mesure de l'impédance, la contre-électrode (28) correspondante étant de préférence prévue dans la surface de recouvrement (9) de la cellule à passage (4) en regard de l'électrode de référence (23).

4. Capteur selon la revendication 3, caractérisé en ce que la contre-électrode (28) du dispositif à électrodes (27) est conçu pour mesurer l'impédance par l'anode (15) de l'électrode à enzymes (13).

5. Capteur selon la revendication 3, caractérisé en ce qu'un perçage (30) opposé coaxialement à la contre-électrode (28) dans le boîtier de la cellule à passage (4), comporte un capteur de température (31) de préférence un capteur à résistance NTC.

6. Capteur selon l'une des revendications 1 à 5, caractérisé en ce que l'électrode à enzymes (13) est un capteur à glucose.
